# EUROPÄISCHE PATENTSCHRIFT

(11) **EP 0 262 186 B1**
(45) Veröffentlichungstag und Bekanntmachung des Hinweises auf die Patenterteilung: **19.07.1995**
(21) Anmeldenummer: 87902058.4
(22) Anmeldetag: 26.03.1987
(51) Int. Cl.: A61B 5/117

(54) **VERFAHREN ZUM ERKENNEN EINES FINGERABDRUCKES**
PROCESS FOR RECOGNIZING A FINGERPRINT
PROCEDE POUR RECONNAITRE UNE EMPREINTE DIGITALE

(30) Priorität: 27.03.1986 DE 3610397
(43) Veröffentlichungstag der Anmeldung: 06.04.1988
(73) Patentinhaber: Sonident Anstalt Liechtensteinischen Rechts, 9490 Vaduz (LI)
(72) Erfinder: BICZ, Wieslaw, D-6500 Mainz 32 (DE)
(74) Vertreter: Funck-Hartherz, Anna-Eleonore, Dipl.-Phys.
(86) Internationale Anmeldenummer: DE8700134
(87) Internationale Veröffentlichungsnummer: WO8705790

(56) Entgegenhaltungen:
- EP-A- 0 048 489
- DE-A- 3 036 912
- US-A- 4 053 228
- Proceedings of the IEEE, Band 67, Nr. 4, April 1979 (IEEE, New York, USA), M. AHMED et al., "Holography and its Application to Acoustic Imaging", Seiten 466-483, siehe Zusammenfassung; Seiten 472-480, Abschnitte "Applications of Holography" und "System Concept and Embodiment"

## Beschreibung

Die Erfindung betrifft ein Verfahren zum Erkennen von Fingerabdrücken und ähnlichen Hautoberflächenstrukturen mit Hilfe von Ultraschall und einer Vorrichtung zur Durchführung eines solchen Verfahrens. Geräte zum Erkennen von Fingerabdrücken sind bereits in verschiedenen Ausführungen bekannt, beziehungsweise vorgeschlagen worden. So gibt es beispielsweise Systeme, bei denen die einmal erfaßten Fingerabdrucksmerkmale in einem zentralen Computer gespeichert sind, aus dem die Merkmale bei Bedarf zum Vergleich mit dem tatsächlichen Fingerabdruck, der durch ein Erfassungsgerät erhalten wird, abgerufen werden können. Derartige Systeme können jedoch aus Datenschutzgründen auf Bedenken stoßen. Es sind daher schon Systeme bekannt geworden, bei denen die Merkmale eines Fingerabdruckes in eine Art Identifikationskarte aufgezeichnet sind, welche der Inhaber der Karte selbst zum Vergleich mit seinem entsprechenden Finger unmittelbar in ein Erkennungsgerät eingibt.

Die Durchführung des Vergleiches erfolgt bei den bekannten Systemen auf unterschiedliche Art und Weise. So sind rein elektronische Verfahren bekannt, aber auch solche, bei denen zu einem großen Teil die optische Erfassung herangezogen wird und nur der endgültige Vergleich der Daten elektronisch erfolgt. Bei rein elektronischen Erfassungsverfahren kann die Abtastung des Fingerabdruckes beispielsweise durch kapazitive oder andere entsprechend kleine Sensoren durchgeführt werden, die in Matrixform angeordnet sind. Für die optische Erfassung können Prismen dienen, an die die Oberfläche der Finger angedrückt wird. Hierbei ändert beispielsweise die Prismenoberfläche ihr Reflexionsverhalten an denjenigen Stellen, an denen das angrenzende Medium nicht mehr Luft, sondern die an der Prismenfläche anliegenden, erhabenen Stellen der Fingeroberfläche sind.

Alle diese bekannten Geräte haben den gemeinsamen Nachteil, daß sie verhältnismäßig kompliziert aufgebaut sind und deshalb keine billigen Ausführungsformen erlauben, die großserienmäßig in kleine Erfassungsgeräte eingebaut werden könnten. Viele dieser verhältnismäßig komplizierten und daher oft auch relativ genauen Erfassungsgeräte erlauben ohne weiteres die Anbindung an eine Datenverarbeitungs- und -erfassungsanlage. Dieser Vorteil ist jedoch nur in bestimmten Fällen erforderlich, und die zentrale Erfassung der Fingerabdruckdaten kann, wie bereits oben erwähnt, auf datenschutzrechtliche Bedenken stoßen.

Aus der DE-A 30 36 912 ist ein Verfahren zum Identifizieren von Personen mittels Fingerabdruckinformationen bekannt, bei der der Fingerabdruck zunächst auf einem thermoplastischen Film gespeichert wird und das so erzeugte Profilbild von einer optischen Strahlungsquelle beleuchtet wird. Das Licht muß anschließend in 30 Kanäle aufgeteilt werden, eine Transformationslinse sowie ein holographisches Filter passieren. Diese Vorrichtung ist äußerst komplex und damit störanfällig sowie kostenintensiv in der Herstellung.

Die EP-A 0 048 489 und die US-A 4 053 228 beschreiben Vorrichtungen zum Untersuchen von Fingerabdrücken, die im Wege der optischen Abtastung und unter Verwendung eines optischen Hologramms die Identifikation der Fingeroberflächen vornehmen. Die bekannten optischen Verfahren haben ferner den Nachteil, daß eine sehr große Datenmenge anfällt, die aufwendige stationäre Datenverarbeitungsanlagen erforderlich machen, weil das optisch erfaßte Bild nicht so leicht komprimierbar ist.

Bei den optische Verfahren, bei denen eine Fingeroberfläche mit einem Hologramm direkt korreliert wird, wird bei Licht mit Wellenlängen über ca. 1 »m gearbeitet. Die Struktur des Fingers liegt aber im Bereich von 1/2 mm, sie ist also ca. 500 x grösser als die Wellenlänge des Lichts, was dazu führt, daß die Streuung des Lichts an einer solchen Struktur äußerst gering ist und damit nicht erfaßbar.

Obwohl in der EP-A 0 048 489 auch das Ultraschallverfahren angesprochen wird, ist lediglich durch einen Austausch der optischen Komponenten durch entsprechende Ultraschallkomponenten eine Identifikation von Fingerobenflächenstrukturen nicht möglich. Über die Ultraschallkomponenten sind keine näheren Angaben gemacht und deshalb eine Ausführbarkeit nicht gegeben.

Ausgehend von dem genannten Stand der Technik und der EP-A-0 048 489 besteht die zu lösende Aufgabe darin, die besonderen Eigenschaften von Ultraschall zu nutzen, um ein Verfahren und eine vorteilhafte Vorrichtung zu schaffen, die eine sichere und wiederholbare Erkennung von Fingerabdrücken und ähnlichen Hautoberflächenstrukturen ermöglichen.

Diese Aufgabe wird erfindungsgemäß durch ein Verfahren nach dem Kennzeichen des Patentanspruchs 1 gelöst. Durch die Verwendung von Ultraschallwellen gegenüber Lichtwellen bei der Fingerabdruckerkennung ergeben sich folgende Vorteile:
1. Durch die Tatsache, daß der Schall sich in Luft etwa fünfmal langsamer ausbreitet als in Wasser, dem in etwa auch die meisten Teile des menschlichen Körpers entsprechen, und es überdies keine Schwierigkeiten bereitet, als Auflageplatte für den Finger ein Material zu finden, welches ähnliche Schallausbreitungseigenschaften wie Wasser hat, läßt sich erreichen, daß an der Übergangsstelle der Haut und der Auflagefläche die Schallwellen ohne weiteres an den Stellen mit etwa gleicher Geschwindigkeit durchgehen, an denen die erhabenen Stellen der Haut des Fingers die Auflagefläche unmittelbar berühren, und dort reflektiert werden, wo sich die Mulden der Fingerstruktur befinden, sich also eine Luftschicht zwischen der Auflagefläche und der Haut befindet. Ohne zusätzliche Maßnahmen wird hierdurch ein guter Kontrast der Fingerabdruckmerkmale erreicht.
2. Da die Schallwellen sich langsamer fortpflanzen und länger sind als Lichtwellen, läßt sich die Analyse der Interferenzmuster auf einfache Weise elektronisch durchführen.
3. Im Gegensatz zu Licht, bei dem holographische Aufzeichnungen nur durch Verwendung von Laserlicht möglich sind, gibt es für den Fall der Verwendung der akustischen Holographie keine Probleme mit der Kohärenz der Wellen.
4. Ultraschallerzeuger für Frequenzen im Bereich von 10 - 15 Megahertz, die beim vorliegenden Verfahren bevorzugt werden, sind einfach und billig und lassen sich sehr präzise steuern, was zum Beispiel dazu führt, daß man ohne jegliche Schwierigkeiten Reflexe und Streuwellen eliminieren kann. Das gilt auch für die Schallempfänger. Es ist aber darüber hinaus möglich, das gleiche Element als Schallerzeuger und als Schallempfänger zu verwenden.
5. Im Falle der Anwendung akustischer Hologramme lassen sich diese wesentlich leichter als optische Hologramme berechnen und produzieren, weil die Wellenlänge größer ist.
6. Die Wellenlänge läßt sich der Struktur der Oberfläche des Fingers anpassen, was dazu führt, daß der Finger sich als ein aktives Element bei der Erkennung ausnutzen läßt.

Die Erfassung von Fingerabdrücken mittels Schallwellen, insbesondere solcher Wellen im Ultraschallbereich, läßt sich gemäß der Erfindung auf zwei unterschiedliche Arten durchführen.

Am anschaulichsten gestaltet sich die Abtastung der Fingeroberfläche punkt- bzw. zeilenweise mittels eines fokussierten Ultraschallstrahles im Scanning-Verfahren. Dabei kann die erforderliche Relativbewegung zwischen dem Ultraschallstrahl und der Fingeroberfläche durch eine mechanische Bewegung der Fingerauflage und/oder eine Bewegung der Ultraschallquelle erreicht werden. Auch ist es möglich, den Ultraschallstrahl durch das Schwenken einer Ablenkeinrichtung im Wellenweg zu bewegen. Eine Strahlbewegung ohne mechanisch bewegte Teile läßt sich durch gesteuerte Phasenverschiebung in der zu bündelnden Wellenfront erreichen. Das durch den Ultraschallstrahl abgetastete Intensitäts- bzw. Kontrastraster ist dann einer computergestützten Auswertung für den Vergleich mit den gespeicherten Daten zu unterwerfen.

Eine weitere Möglichkeit vergleichbaren Erfassens von Fingerabdrücken mittels Ultraschall besteht darin, daß die reflektierte Schallwellenstruktur physikalisch und auch rechnerisch einer Bildtransformation, z. B. einer Fouriertransformation unterworfen wird, um eine für Auswertungs- und Vergleichzwecke einfachere Struktur der zu speichernden Daten zu erhalten.

Die Signale der transformierten Struktur können durch einen Matrixempfänger oder einen Scanner in einen Computer übertragen werden, der den Vergleich mit den aus einer entsprechenden Transformation hervorgegangenen, gespeicherten Daten vornimmt.

Schließlich kann auch die akustische Holographie zur Fingerabdruckerkennung herangezogen werden.

Die für das Erkennungsverfahren verwendeten gespeicherten akustischen Hologramme der Fingeroberfläche können eine den Merkmalen des Fingers entsprechende Oberflächenreliefstruktur aufweisen, an der die Schallwellen unterschiedlich reflektiert werden. Solche Hologramme, bei denen die Merkmalsstruktur sichtbar in die Oberfläche eingeprägt ist, sind jedoch aus Sicherheitsgründen wenig geeignet. Es sind daher akustische Hologramme vorzuziehen, die von außen sichtbar eine ebene Oberfläche aufweisen, unterhalb derer jedoch Materialien unterschiedlicher Schallfortpflanzungsei genschaften verwendet sind. Bei Hologrammen, die von den Ultraschallwellen durchstrahlt werden, können Bereiche unterschiedlicher akustischer Transparenz verwendet werden.

Der Verlauf der Schallwellen und die Anordnung der einzelnenen Elemente im Erkennungssystem können unterschiedlich gewählt werden. So ist es beispielsweise möglich, die vom Ultraschallerzeuger beziehungsweise der Ultraschallquelle kommenden Schallwellenfronten zuerst durch den das akustische Hologramm enthaltenden Aufzeichnungsträger hindurchzusenden, um sie dann durch die Fingerauflageplatte hindurch auf die Fingergrenzfläche auftreffen zu lassen, von der die Schallwellen dann der Fingeroberflächenstruktur entsprechend unterschiedlich reflektiert und einem Ultraschallrasterempfänger zugeführt werden. Es ist aber auch die umgekehrte Anordnung möglich, bei der die Schallwellen zuerst auf die Fingergrenzfläche gestrahlt und die von dort reflektierten Wellen dann durch das Hologramm hindurchgeschickt werden, um austrittseitig dann auf den Rasterempfänger aufzutreffen.

Eine weitere Methode besteht darin, den das akustische Hologramm enthaltenden Aufzeichnungsträger unmittelbar als Fingerauflageplatte zu verwenden. Bei dieser Anordnung werden die Schallwellen von der der Fingerauflagefläche abgewandten Seite des akustischen Hologrammes gegen dieses gerichtet und die von dem Hologramm und der Fingergrenzfläche reflektierten Wellen in einem Empfänger aufgenommen.

Schließlich ist auch eine Anordnung möglich, bei der die von der Schallquelle kommenden Schallwellen gleichzeitig einerseits auf die Fingergrenzfläche und andererseits auf ein reflektierend arbeitendes Hologramm gerichtet werden. Die von beiden Flächen reflektierten Schallwellen werden dann dem Rasterempfänger zugeführt. Eine Variante dieser Anordnung besteht darin, daß Schallerzeuger und Schallempfänger im wesentlichen in der gleichen Ebene liegen, und die Schallwellen in Anordnung eines gleichseitigen Dreiecks von dort auf die Fingergrenzfläche, von hier auf das Hologramm und zurück zum Empfänger und gleichzeitig in umgekehrter Richtung gerichtet werden.

Für den Fall, daß Schallerzeuger und Empfänger das gleiche Bauteil sind, wird intermittierend im Impulsverfahren gearbeitet, während bei getrenntem Empfänger der Schallerzeuger auch mit kontinuierlicher Schallausstrahlung arbeiten kann.

Für die Arbeitsweise des Systems ist es von besonderem Vorteil, wenn sich die gesamte Meßanordnung insoweit der Verlauf der Ultraschallwellen betroffen ist und zwar im wesentlichen der Ultraschallerzeuger und der Empfänger in Flüssigkeit, zweckmäßigerweise in Wasser befinden. Die Flüssigkeit grenzt dann in jedem Fall bis an die Rückseite der Fingerauflageplatte, die, wie schon oben erwähnt, aus einem Material bestehen soll, dessen Schallausbreitungsgeschwindigkeit derjenigen der Flüssigkeit im wesentlichen entsprechen soll um einen zusätzlichen Grenzflächenübergang zu vermeiden. Statt einer Flüssigkeit kann beispielsweise auch ein festes, vorzugsweise Kunststoffmaterial mit entsprechenden Eigenschaften verwendet werden, in das die Meßanordnung eingebettet ist, oder an das der Schallerzeuger bzw. Empfänger angekoppelt ist.

### Kurze Beschreibung der Zeichnungen

Im folgenden wird das erfindungsgemäße Verfahren anhand der beigefügten Zeichnungen im einzelnen noch näher erläutert. In den Zeichnungen stellen dar:
- Fig.1:: Eine schematische Darstellung der Verhältnisse, wie Ultraschallwellen an der Grenzfläche zwischen einer Fingeroberfläche und einer ebenen Auflagefläche reflektiert werden;
- Fig.2:: eine erste Anordnung für den Schallwellenverlauf bei der Erkennung von Fingerabdrücken mittels akustischer Holographie;
- Fig.3:: eine zweite Anordnungsmöglichkeit für den Verlauf der Schallwellen bei der holographischen Methode;
- Fig.4:: eine dritte Anordnungsmöglichkeit für den Verlauf der Schallwellen bei der holographischen Methode;
- Fig.5:: eine vierte Anordnungsmöglichkeit für den Verlauf der Schallwellen bei der holographischen Methode;
- Fig.6:: eine fünfte Möglichkeit für die Anordnung des Verlaufs der Schallwellen bei der holographischen Methode;
- Fig.7:: die schematische Anordnung der Abtastung einer Fingeroberfläche mittels fokussierten Ultraschalls; und
- Fig.8:: die schematische Anordnung für eine physikalische Transformation einer von einer Fingeroberfläche reflektierten Ultraschallstruktur.

### Wege zur Ausführung der Erfindung

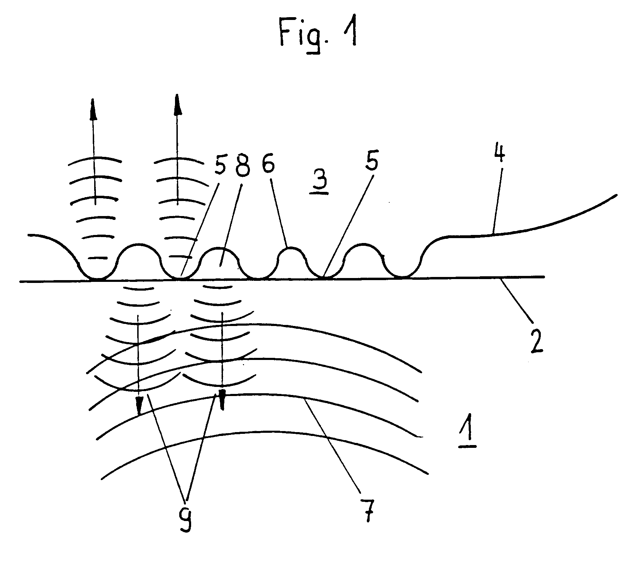

In Figur 1 ist ohne ihre untere Begrenzung eine Auflageplatte (1) dargestellt, die eine ebene Oberfläche (2) aufweist. Anliegend an die Oberfläche (2) befindet sich ein Teil (3) eines menschlichen Fingers. Die Oberfläche (4) des Fingers weist erhabene Stellen (5) und vertiefte Stellen (6) auf. Durch die Auflageplatte (1), deren Material möglichst eine Schallausbreitungsgeschwindigkeit hat, die in etwa derjenigen von Wasser entspricht, wird eine Front von Ultraschallwellen (7) im Frequenzbereich von 10 bis 15 Megahertz geschickt, die auf die Grenzfläche zwischen Auflageplatte (1) und Fingeroberfläche (4) auftritt. An den Stellen, an denen sich die erhabenen Bereiche (5) der Fingeroberfläche (4) in unmittelbarer Berührung mit der Oberfläche (2) der Auflageplatte (1) befinden, treten die Ultraschallwellen in starkem Maße durch die Grenzfläche zwischen Finger und Auflageplatte hindurch und pflanzen sich innerhalb des Fingers mit im wesentlichen gleicher Geschwindigkeit weiter fort, wenn die Schallausbreitungsgeschwindigkeit in der Auflageplatte (1) im wesentlichen derjenigen innerhalb des Fingers entspricht. Im Bereich der Vertiefungen (6) der Fingeroberfläche dagegen, in denen sich hinter der Oberfläche (2) der Auflageplatte (1) Lufträume (8) befinden, werden die Schallwellen wegen der wesentlich geringeren Ausbreitungsgeschwindigkeit in Luft in Form von örtlichen Wellenfronten (9) reflektiert. Diese Wellenfronten (9) bilden eine bestimmte Interferenzstruktur, die von einem in den Ausbreitungsgang der Wellenfronten (9) angebrachten Rasterempfänger erfaßt werden.

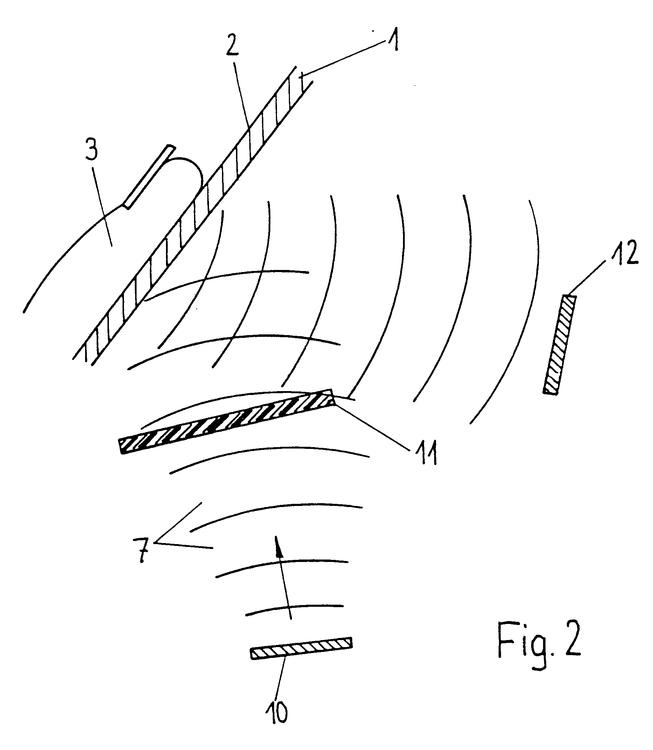

In den folgenden Figuren sind verschiedene Anordnungen für den Schallwellenverlauf bei dem erfindungsgemäßen System dargestellt. Bei der Anordnung gemäß Figur 2 treffen die von einem Ultraschallerzeuger (10) erzeugten Schallwellen (7) zuerst auf eine das akustische Hologramm des Fingerabdruckes enthaltende Karte (11) auf. Die durch die Karte (11) hindurchtretenden Schallwellen erreichen sodann die Grenzfläche zwischen der Auflageplatte (1) und dem Finger (3). In der Richtung der von dieser Grenzfläche reflektierten Schallwellen befindet sich ein Rasterempfänger (12), mit dem die erhaltenen Interferenzmuster erfaßt werden.

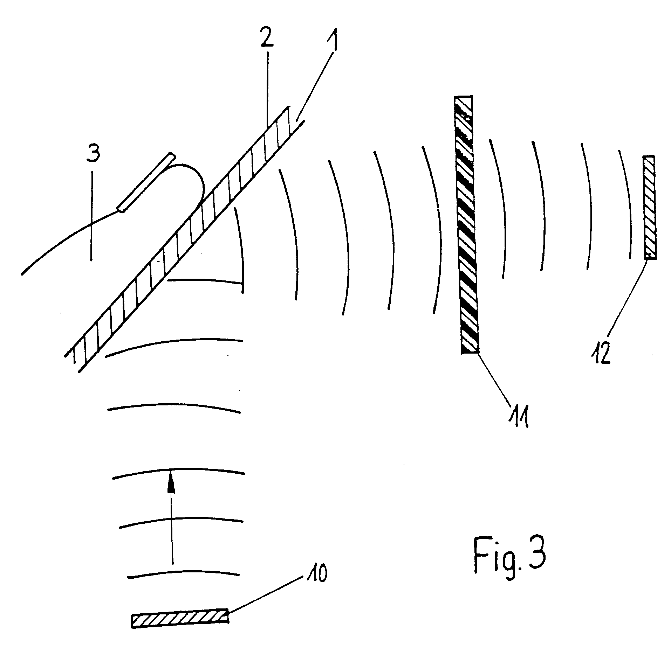

Die Anordnung nach Figur 3 unterscheidet sich von derjenigen nach Figur 2 nur dadurch, daß die Schallwellen zuerst auf die Grenzfläche zwischen Finger und Auflageplatte auftreffen, und erst die reflektierten Schallwellen durch die das akustische Hologramm enthaltende Karte (11) hindurchtreten.

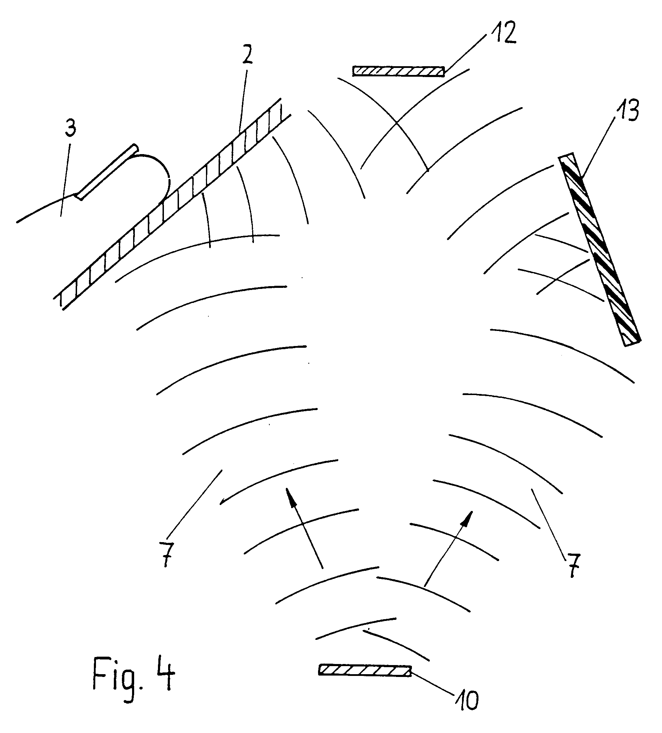

In Abbildung 4 ist eine Anordnung dargestellt, bei der die Schallwellen (7) der vom Ultraschallerzeuger (10) erzeugten Wellenfront unmittelbar sowohl auf die Grenzfläche zwischen Finger (3) und Auflagefläche (2) als auch gegen eine ein akustisches Hologramm enthaltende Karte (13) gesandt werden, an der jedoch im Unterschied zu den beiden zuvor beschriebenen Ausführungsformen die Schallwellen ebenfalls reflektiert werden. Beide reflektierten Wellen interferieren und werden von dem Empfänger (12) erfaßt. Der gesamte Schallwellenverlauf findet in einer Flüssigkeit statt, deren Schallausbreitungsgeschwindigkeit mit derjenigen der Fingerauflageplatte im wesentlichen übereinstimmt.

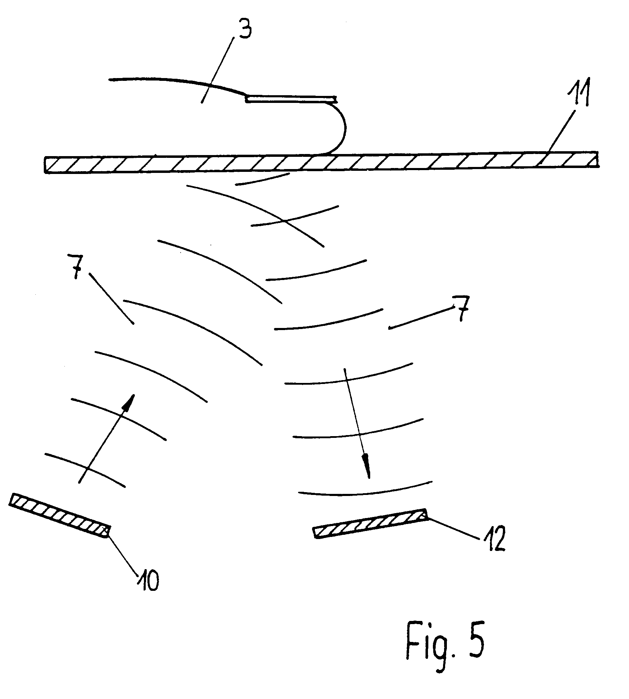

Bei der Anordnung gemäß Figur 5 wird die das akustische Hologramm enthaltende Karte (11) unmittelbar als Auflagefläche für den Finger (3) verwendet. Die Schallwellen (7) durchlaufen die Karte (11), werden zum Teil an der Grenzfläche zwischen Finger und Karte reflektiert, und die reflektierten Wellen werden von dem Empfänger (12) erfaßt.

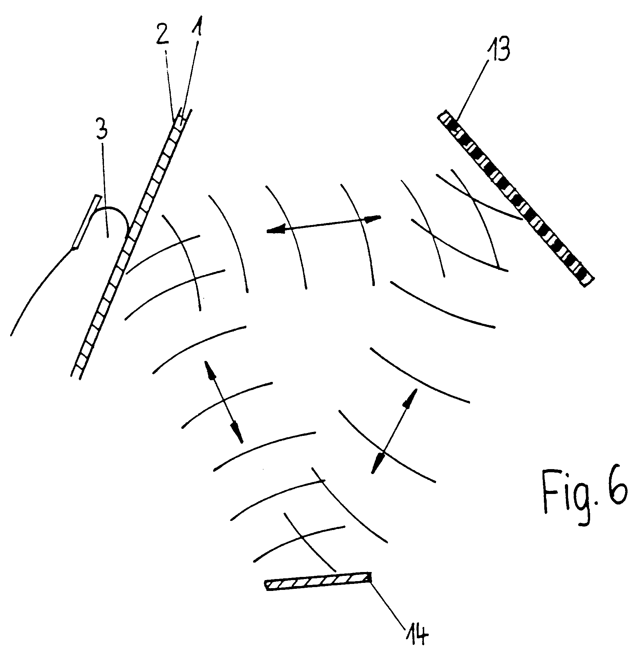

Eine letzte Ausführungsform ist schließlich in Figur 6 dargestellt. Dort sind Schallerzeuger und Empfänger zu einer Einheit (14) miteinander kombiniert. Diese Einheit sendet Schallwellen sowohl gegen die Grenzfläche zwischen Auflagefläche (2) und Finger (3) aus als auch gegen die ein akustisches Reflexionshologramm enthaltende Karte (13). Durch die symmetrische Anordnung in Form eines gleichseitigen Dreieckes werden einerseits Schallwellen von der Fingergrenzfläche zur Karte (13) und andererseits auch von dieser zur Fingergrenzfläche reflektiert, wobei die dort jeweils reflektierten Schallwellen wieder zu dem Sender/Empfänger zurück reflektiert werden.

Die Rasterempfänger für die Ultraschallwellen können beispielsweise aus einem Raster kleiner piezoelektrischer Empfängerelemente bestehen, in denen der jeweils auf eine Rastereinheit auftreffende Schalldruck in ein elektrisches Signal umgewandelt wird. Das erhaltene Raster an elektrischen Signalen wird dann einer nicht dargestellten elektronischen Auswerteinheit zugeführt, die den erforderlichen Vergleich auf elektronische Weise durchführt.

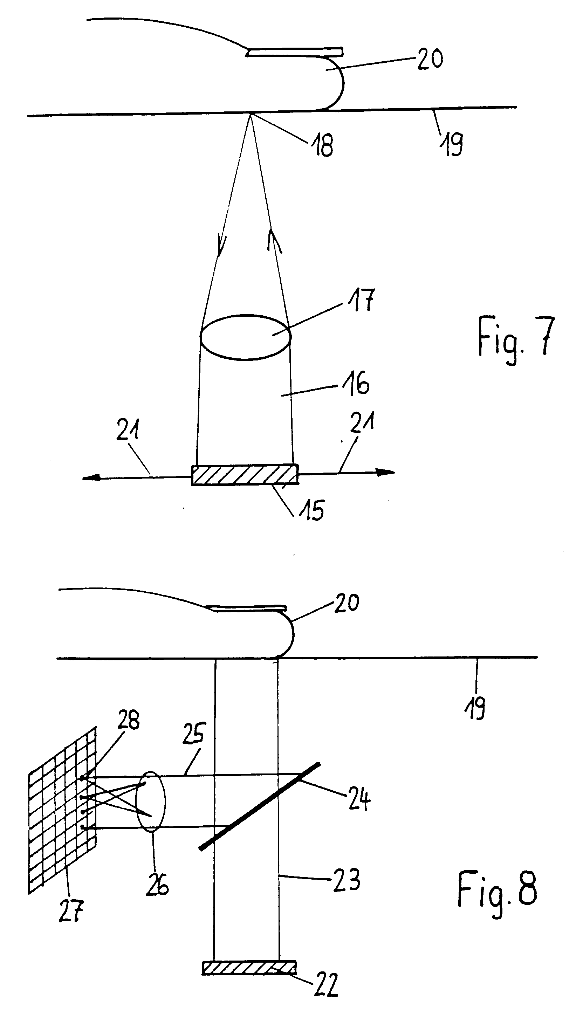

In Fig. 7 ist die schematische Anordnung für die Antastung einer Fingeroberfläche mittels gebündelten Ultraschalls dargestellt. Ein kombinierter Ultraschallsender und -empfänger (15) sendet eine Ultraschallwellenfront (16) aus, die durch eine Schallwellen fokussierende Linsenanordnung (17) auf einen Punkt (18) fokussiert wird, welcher auf die Grenzfläche zwischen einer Kontaktfläche (19) und der Oberfläche eines Fingers (20) einjustiert wird. Der vom Punkt (18) reflektierte Schall wird wieder dem Sender/Empfänger (15) zugeführt, was durch die entgegengesetzten Pfeile im Strahlenweg angedeutet ist. Der Sender/Empfänger (15) ist für intermittierendes Senden und Empfangen eingerichtet, so daß die Abtastung impulsweise erfolgt. Die Pfeile (21) sollen andeuten, daß die Schallquelle (15) zum Abtasten der Fingeroberfläche hin- und herbewegt wird.

Fig. 8 veranschaulicht eine Erfassungsanordnung mit physikalischer Transformation der reflektierten Wellenstruktur. Ein Ultraschallsender (22) sendet eine Wellenfront (23) aus, die durch einen in dieser Richtung durchlässigen Spiegel (24) hindurch auf die Grenzfläche zwischen der Kontaktfläche (19) und der Oberfläche des Fingers (20) gerichtet ist. Die von der Grenzfläche reflektierte Wellenfront wird an dem Spiegel (24) zumindest teilweise umgelenkt und als Wellenfront (25) einem physikalischen Transformationselement (26) in Form einer akustischen Linsenanordnung zugeleitet. Auf einer Auffangfläche (27), beispielsweise einer akustisch/elektrischen Wandlermatrix entsteht ein durch die Transformation vereinfachtes Strukturbild in Form eines Punkterasters (28), welches einer vereinfachten Auswertung, d.h. einem Vergleich mit einem auf entsprechende Art und Weise erzeugten, gespeicherten Merkmalsraster zugänglich ist.

## Patentansprüche

1. Verfahren zum Erkennen von Fingerabdrücken und ähnlichen Hautoberflächenstrukturen mit Hilfe von Ultraschall,
dadurch gekennzeichnet,
- daß der Finger- oder Hautbereich auf eine, eine glatte Oberfläche (2) aufweisende, für Ultraschallwellen transparente Auflageplatte (1) aufgelegt wird, wobei sich zwischen der Auflagefläche (2) und den Vertiefungen der Finger- oder Hautoberfläche (4) Luft befindet,
- daß die Finger- oder Hautoberfläche (4) mit Ultraschall mit einer Frequenz von 10 - 15 Megahertz beschallt wird, wobei die von der Ultraschallquelle (10, 14, 15, 22) ausgehenden Schallwellen (7, 16, 23) und die von der Finger- oder Hautoberfläche (4) reflektierten Schallwellen (9) durch eine Flüssigkeit oder einem Festkörper von der Ultraschallquelle (10, 14, 15, 22) bis zu der Auflageplatte (1) und einem Empfänger (12, 14, 15) geführt werden und wobei die verwendeten Materialien der Auflageplatte (1) und der Flüssigkeit oder des Festkörpers den Schallausbreitungseigenschaften innerhalb die Haut entsprechende Schallausbreitungseigenschften besitzen,
- und daß (Alternative A) die Schallwellen (7) und/oder die reflektierten Schallwellen (9) für einen Merkmalsvergleich ein die Merkmale eines Finger- oder Hautabdrucks enthaltendes akustisches Hologrammen (11, 13), das sich in einem Aufzeichnungsträger befindet, durchlaufen oder von diesem Hologramm reflektiert werden, und die von der Finger- oder Hautoberfläche (4) und dem Hologramm (11, 13) reflektierten oder transmittierten Schallwellen einen Rasterempfänger (12, 14) zugeführt werden zum Umwandeln von Ultrschallsignalen in elektrische Signale, die in einer elektronischen Auswerteinheit ausgewertet werden,
- oder daß (Alternative B) die von der Finger- oder Hautoberfläche (4) reflektierten Schallwellen mittels fokussierten Schalls in geeignete Auswertmerkmale transformiert und einen Ultraschallempfänger (15, 27) zügeführt werden und anschließend mit einer auf einem Aufzeichnungsträger gespeicherten Merkmalsstruktur verglichen werden.

2. Verfahren nach Alternative B des Anspruchs 1,
**dadurch gekennzeichnet**,
daß die Ultraschallwelle (16) zu einem Schallstrahl (18) fokussiert wird und eine Relativbewegung zwischen dem fokussierten Schallstrahl (18) und der Fingeroberfläche (4) durch eine Bewegung (21) des Fingers (20), der Schallquelle (15) und/oder eines im Wege des Schallstrahles befindlichen Ablenkelements erzeugt wird.

3. Verfahren nach Alternative B des Anspruchs 1,
**dadurch gekennzeichnet**,
daß die Ultraschallwelle (16) zu einem Schallstrahl (18) fokussiert wird und eine Bewegung des Endpunktes des fokussierten Schallstrahles durch Phasenverschiebungen innerhalb der erzeugten Schallwellenfront erfolgt.

4. Verfahren nach Alternative A des Anspruchs 1,
**dadurch gekennzeichnet**,
daß für den Merkmalsvergleich die von einem Ultraschallerzeuger (10) erzeugten Schallwellen (7) gegen das akustische Hologramm (11) gerichtet, die durch das Hologramm (11) hindurchtretenden Schallwellen auf die Fingeroberfläche (4) gerichtet und die von dieser reflektierten Schallwellen von dem Rasterempfänger (12) erfaßt werden.

5. Verfahren nach Alternative A des Anspruch 1,
**dadurch gekennzeichnet**,
daß für den Merkmalsvergleich die von einem Ultraschallerzeuger (10) erzeugten Schallwellen (7) zuerst auf die Fingeroberfläche (4) gerichtet und mit den dort reflektierten Schallwellen das akustische Hologramm (11) durchstrahlt wird und die aus diesem austretenden Schallwellen von dem Rasterempfänger (12) erfaßt werden.

6. Verfahren nach Alternative A des Anspruchs 1,
**dadurch gekennzeichnet**,
daß die von einem Ultraschallerzeuger (10) erzeugten Schallwellen (7) sowohl auf die Fingeroberfläche (4) als auch auf das akustische Hologramm (13) gerichtet und die von beiden reflektierten Schallwellen von dem Rasterempfänger (12) erfaßt werden.

7. Verfahren nach Alternative A des Anspruchs 1,
**dadurch gekennzeichnet**,
daß das Hologramm (11) als Auflageplatte (1) für die Fingeroberfläche (4) dient.

8. Verfahren nach Alternative A des Anspruchs 1,
**dadurch gekennzeichnet**,
daß die von einem Ultraschallerzeuger (14) erzeugten Schallwellen (7) in symmetrischer Anordnung eines gleichseitigen Dreieicks zuerst auf die Fingerober` fläche (4), die von dieser reflektierten Schallwellen auf das Hologramm (13) und die von dort reflektierten Schallwellen zurück auf einen Matrixempfänger (14) in der Ebene des Schallerzeugers wie auch im entgegengesetzten Verlauf gerichtet werden.

9. Verfahren nach Anspruch 8,
**dadurch gekennzeichnet**,
daß der Schallerzeuger (14) gleichzeitig als Empfänger verwendet wird.

10. Vorrichtung zur Durchführung des Verfahrens nach einem der Ansprüche 4 bis 9,
**gekennzeichnet durch**
einen Ultraschallerzeuger (10, 14) für Frequenzen von 10 bis 15 Megahertz, eine für Ultraschallwellen transparente Auflageplatte (1) mit einer glatten Oberfläche (2) zum Auflegen eines Fingers (3), eine Flüssigkeit oder einen Festkörper zwischen dem Ultraschallerzeuger (10, 14), dem Ultraschallempfänger (12, 14, 15) und der Auflageplatte, wobei sich die Flussigkeit oder der Festkörper bis an die Auflageplatte (1) erstreckt und wobei die Auflageplatte und die Flüssigkeit oder der Festkörper aus Materialien bestehen, die eine der Schallausbreitungsgeschwindigkeit innerhalb des Fingers entsprechende Schallausbreitungsgeschwindigkeit besitzen, ein akustisches Hologramm (11, 13) im einem Aufzeichnungsträger, welches die Merkmale eines Fingerabdruckes enhält, einen Rasterempfänger (12, 14) zum Umwandeln von Ultraschallenergie in elektrische Signale und eine elektronische Auswerteinheit zum Auswerten der Signale.

11. Vorrichtung nach Anspruch 10,
**dadurch gekennzeichnet**,
daß das Material der Auflageplatte (1) eine größere, vorzugsweise eine fünffach größere Schallgeschwindigkeit als Luft aufweist.

12. Vorrichtung nach einem der Ansprüche 10 oder 11,
**dadurch gekennzeichnet**,
daß das akustische Hologramm (11) mittels Ultraschallwellen (7) durchstrahlbar ist.

13. Vorrichtung nach einem der Ansprüche 10 bis 12,
**dadurch gekennzeichnet**,
daß der Ultraschallerzeuger (10) gleichzeitig als Schallempfänger (14) ausgebildet ist.

14. Vorrichtung nach Anspruch 10,
**dadurch gekennzeichnet**,
daß zusätzliche schallfokussierende und schallreflektierende Elemente vorgesehen sind.

15. Vorrichtung nach Anspruch 1,
**dadurch gekennzeichnet**,
daß die von der Fingeroberfläche (4) reflektierte Schallwellenstruktur durch physikalische Wandlerelemente (26) einer Bildtransformation, vorzugsweise einer Fouriertransformation unterworfen und die transformierte Struktur mittels eines Scanning- oder Matrixempfängers in ein Signalbild (28) umgewandelt wird.

## Claims

1. Method to recognize fingerprints and similar skin surface patterns using ultrasound,
characterized by the following
- the fingertip or the skin area is put on a smooth (2) support piece (1) of material which ultrasonic waves may penetrate, with air between the skin surface (4) and the support piece (1),
- the sender radiates the surface of the fingertip or the skin area (4) with ultrasonic waves of a frequency of 10-15 MHz, which are reflected by the surface of the finger or the skin, said ultrasonic waves are conducted on the way from the sender to the surface of the finger or the skin area and back from it to the receiver through a liquid or a solid and the propertiers of the material of the support piece and the liquid or the solid must be similar to the skin from point of view of ultrasonic waves spreading,
- alternative A
the ultrasonic waves (7) and/or their reflections (9) by the finger or other skin area (4) passing through or are reflected by an acoustic hologram (11,13), which contains the characteristics of the fingerprint or the skin structure and is stored in a data carrier, and are conducted to a scanning receiver (12,14) in order to transform the ultrasonic signals into electrical signals which are evaluated in an electronic evaluation unit for a comparison of the characteristics,
- alternative B
or the ultrasonic waves reflected by the finger or other skin area (4) are transformed into suitable characteristics using focussing and are conducted to an ultrasound receiver (15,27) in order to compare them with the characteristics stored in a data carrier.

2. Method according patent claim 1, alternative B, characterized in that the ultrasonic waves (16) are focussed to an ultrasound beam (18) and between the ultrasound beam (18) and the finger surface (4) a relative motion is executed, this motion may be caused by a movement (21) of the finger (20), of the sender (15) and/or a deflection device.

3. Method according patent claim 1, alternative B, characterized in that the ultrasonic waves (16) are focussed to an ultrasound beam (18) and that the motion of the final point of the ultrasound beam is caused by phase displacement inside the produced front of ultrasonic waves.

4. Method according patent claim 1, alternative A, characterized in that for a comparison of characteristics the ultrasonic waves (7) produced by the sender (10) are conducted to an acoustic hologram (11), the passing waves are conducted to the finger (4) and the waves reflected by the finger surface are received by the scanning receiver (12).

5. Method according patent claim 1, alternative A,
characterized in that for the comparison of characteristics the ultrasonic waves (7) produced by the sender (10) are conducted first to the finger surface (4), are reflected by it and passing the acoustic hologram (11) before being received by the scanning receiver (12).

6. Method according patent claim 1, alternative A,
characterized in that the ultrasonic waves (7) produced by the sender (10) are conducted simultaneously to the finger surface (4) and to the acoustic hologram (13) and the reflections of both are received by the scanning receiver (12).

7. Method according patent claim 1, alternative A,
characterized in that the acoustic hologram (11) is used as support piece (1) for the finger surface (4).

8. Method according patent claim 1, alternative A,
characterized in that in a symetric arrangement in the shape of a equilateral triangle the ultrasonic waves (7) produced by the sender (14) are conducted first to the finger surface (4), the reflections by it are conducted to the acoustic hologram (13) and the reflections by the hologram are conducted to a matrix receiver (14) on the same plane as the sender or the procedure may be done the other way around.

9. Method according patent claim 8,
characterized in that the sender (14) is used simultaneously as receiver.

10. Device to realize the method according the patent claims 4-9,
characterized by the following features
- a transducer (10, 14) for the production of ultrasonic waves (sender) with a frequency of 10-15 MHz,
- a support piece (1) to put the finger (3) with a smooth surface (2) which ultrasonic waves may penetrate,
- a liquid or a solid between the ultrasound sender (10,14), the ultrasound receiver (12,14,15) and the support piece (1) to put the finger,
- the properties of the material of the support piece and the liquid or the solid must be similar to the skin from point of view of ultrasonic waves spreading,
- an acoustic hologram (11,13) stored in a data carrier containing the characteristics of the fingerprint,
- a scanning receiver (12,14) to transform the ultrasonic signals into electrical signals
- and an electronic evaluation unit to evaluate the electrical signals.

11. Device according patent claim 10,
characterized in that the material of the support piece (1) allows the ultrasonic waves a velocity of spreading higher than the air, preferably five times higher.

12. Device according patent claim 10 or 11,
characterized in that the acoustic hologram (11) may be penetrated by ultrasonic waves (7).

13. Device according patent claim 10-12,
characterized in that the transducer (10) serves simultaneously as sender (10) and as receiver (14) of the ultrasonic waves.

14. Device according patent claim 10,
characterized in that supplementary elements are used to focus and/or to reflect the ultrasonic waves.

15. Device according patent claim 1,
characterized in that the structure of the ultrasonic waves reflected by the finger surface (4) is subject to an image transformation, preferably a Fourier transform, using physical transformers (26), and the transformed structure is transformed again to a image of signals (28) using a scanning or a matrix receiver.

## Revendications

1. Procédé à reconnaître les empreintes digitales et les structures semblable de la surface de la peau par ondes ultra-son caractérisé par le fait
- que le doigt ou la peau est posé sur une plaque (1) avec une surface lisse (2) qui laisse passer les ondes ultra-son et ne consiste pas forcément du matériel metallique et ou il reste entre la surface de la plaque(2) et du doigt ou de la peau (4) de l'air,
- que la surface du doigt ou de la peau (4) est exposé à une frequence de 10-15 megahertz des ondes ultra-son (10,14,15,22) qui sont guidé en venant de l'émetteur (7,16,23) ou en réflection (9) de la surface du doigt ou de la peau (4) dans un liquide ou une matière solide dans chaque direction jusqu'à la plaque (1) ou jusqu'au récepteur (12,14,15),
- que le caractère du matériel du liquide ou de la matière solide et de la plaque (1) corresponde au point de vue de la diffusion des ondes ultra-son au caractère de la peau,
- et que (alternative A)
les ondes ultra-son (7) et/ou ses reflexions (9) passent par un hologram acoustique (11,13) ou sont reflechi par ce hologram qui contien les caractéristiques d'un empreint du doigt ou de la peau et qui est deposé dans un support et que ces ondes ultra-son réflechis ou transmis par la surface du doigt ou de la peau (4) et le hologram acoustique (11,13) vont etre dirigé vers un récepteur à réseau (12,14) qui transforme les signaux ultrasoniques en signaux électriques qui sont évalué dans un évaluateur èlectronique,
- ou que (alternative B)
les ondes ultra-son réflechi par la surface du doigt ou de la peau (4) sont transformé par la focalisation du son en caractéristiques adaptés qui sont transmis à un récepteur ultrasonique (15,27) ou ils sont comparé avec la structure caractéristique qui est enregistré sur un support.

2. Procédé après alternative B de la revendication 1 caractérisé par le fait
- que l'onde ultra-son (16) est focalisé à un jet de son (18) et qu'un mouvement rélative entre le jet de son focalisé (16) et la surface du doigt et éxécuté soit il par un mouvement (21) du doigt (20) ou du émétteur (15) et/ou d'un élément qui est installé dans la voie du jet de son pour le détourner.

3. Procédé après alternative B de la revendication 1 caractérisé par le fait
- que l'onde ultra-son (16) est focalisé à un jet de son (18) et qu'un mouvement de la pointe finale du jet de son est causé par un déplacement de la phase dans le front des ondes ultra-son produit.

4. Procédé après alternative A de la revendication 1 caractérisé par le fait
- que pour but de comparaison des caractéristiques les ondes ultra-son (7) produit par un transducteur (10) sont dirigé vers un hologram acoustique (11), les ondes ultra-son qui passent par ce hologram sont dirigés vers la surface du doigt (4) et que les ondes ultra-son reflechis par la surface du doigt vont être enregistré par le récepteur à réseau (12).

5. Procédé après alternative A de la revendication 1 caractérisé par le fait
- que pour la comparaison des caractéristiques les ondes ultra-son (7) produit par un transducteur (10) sont dirigé premièrement vers la surface du doigt (4) et que les ondes ultra-son reflechis par la surface du doigt passent par le hologram acoustique (11) avant d'être enregistré par le récepteur du réseau (12).

6. Procédé après alternative A de la revendication 1 caractérisé par le fait
- que les ondes ultra-son (7) produit par le transducteur (10) sont dirigé simultanément vers la surface du doigt (4) et vers le hologram acoustique (13) et que les ondes ultra-son reflechi par le doigt et par le hologram sont enregistré par le récepteur du reseau (12).

7. Procédé après alternative A de la revendication 1 caractérisé par le fait
- que le hologram acoustique (11) sert à plaque (1) à poser la surface du doigt (4).

8. Procédé après alternative A de la revendication 1 caractérisé par le fait
- que les ondes ultra-son (7) produit par un transducteur (14) sont dirigé dans un arrangement symétrique dans la forme d'un triangle equilatéral premièrement vers la surface du doigt (4), ensuite les ondes ultrason reflechi de la vers le hologram acoustique (13) et ensuite les ondes ultra-son reflechi de la vers un récepteur à matrice ou dans le sens invers.

9. Procédé après revendication 8 caractérisé par le fait
- que le transducteur (14) sert simultanément à émetteur et récepteur des ondes ultra-son.

10. Dispositif pour la réalisation des procédés après les revendications 4-9 caractérisé par
- un transducteur (10,14) pour la production des ondes ultra-son des frequences de 10-15 megahertz,
- une plaque (1) avec une surface lisse (2) qui ne consiste pas forcément du matériel metallique et qui laisse passer les ondes ultra-son à la pose du doigt (3),
- un liquide ou un solide entre le transducteur (10,14) émetteur, le transducteur récepteur (12,14,15) et la plaque à pose-doigt (1), le liquide ou solide et la plaque consistant des matériaux qui permettent aux ondes ultra-son la même vitesse d'extension comme dans l'interieur du doigt,
- un hologram acoustique (11,13) dans un support contenant les caractéristiques d'un empreint du doigt,
- un récepteur du réseau (12,14) pour la transformation des signaux ultrasoniques en signaux électriques
- et un évaluateur électronique pour evaluer les signaux électriques.

11. Dispositif après la revendication 10 caractérisé par le fait
- que le matériel de la plaque (1) à pose-doigt permette aux ondes ultra-son une vitesse d'extension plus grand que l'air, de préférence cinque fois plus grande.

12. Dispositif après le revendications 10 ou 11 caractérisé par le fait
- que l'hologram acoustique (11) permette le passage des ondes ultra-son.

13. Dispositif après les revendications 10-12 caractérisé par le fait
- que le transducteur (10) sert simultanément à émetteur (10) et récepteur (14) des ondes ultra-son.

14. Dispositif après revendication 10 caractérisé par
- mesures complementaires pour focalisation et/ou réflexion des ondes ultra-son.

15. Dispositif après revendication 1 caractérisé par le fait
- que la structure des ondes ultra-son réflechi par la surface du doigt (4) est soumis à l'aide des éléments physiques de transformation (26) à une transformation d' images, de préférence une transformation après Fourier, et la structure transformé est à l'aide d'un récepteur à scanning ou à matrice de nouveau transformé en image de signaux (28).
